# EUROPEAN PATENT SPECIFICATION

(11) **EP 2 658 457 B1**
(45) Date of publication and mention of the grant of the patent: **28.11.2018**
(21) Application number: 11818917.4
(22) Date of filing: 28.12.2011
(51) Int. Cl.: A61B 17/32, A61B 17/3211

(54) **TENOTOME FOR HIGH PRECISION INCISION OF THE TRANSVERSE CARPAL LIGAMENT WITHOUT DAMAGE TO THE ADJOINING TISSUE**
TENOTOME FÜR HOCHPRÄZISE INZISION DES LIGAMENTUM CARPI TRANSVERSUM OHNE BESCHÄDIGUNG DES ANGRENZENDEN GEWEBES
TÉNOTOME POUR UNE INCISION DE HAUTE PRÉCISION DU LIGAMENT CARPIEN TRANSVERSE SANS DOMMAGE AU TISSU ADJACENT

(30) Priority: 30.12.2010 IT PI20100032 U
(43) Date of publication of application: 06.11.2013
(73) Proprietor: Stampacchia, Marcello, 48013 Brisighella (IT); Stampacchia, Stefano, 00139 Roma (IT)
(72) Inventor: Stampacchia, Marcello, 48013 Brisighella (IT); Stampacchia, Stefano, 00139 Roma (IT)
(74) Representative: Lualdi, Lorenzo
(86) International application number: PCT/EP2011/074149
(87) International publication number: WO 2012/089767

(56) References cited:
- WO-A2-2010/030872
- US-A1- 2007 288 043
- US-A1- 2008 109 021
- US-A1- 2009 048 620

## Description

### FIELD OF THE INVENTION

This invention relates to an instrument for the surgical treatment of carpal tunnel syndrome and in particular to a surgical instrument that allows incision of the transverse carpal ligament, in order to decompress the carpal tunnel safely and with precision.

### BACKGROUND OF THE INVENTION

As sector operators are well aware, Carpal Tunnel Syndrome (CTS) is a symptom of entrapment neuropathy and is caused by compression of the median nerve of the wrist as it passes through the aforementioned carpal tunnel, which contains nerve structures (the median nerve), vascular structures and muscle tendons that flex the fingers. For over 50 years a surgical procedure has been used to treat this dysfunction. Different instruments and surgical methods have been developed in this period of time. One such method consists of an "open" surgical procedure. In this case, a longitudinal incision is made, parallel to the median nerve, which divides the transverse ligament into two separate sections. Several problems may arise in connection with this 15-20 minute procedure, both during the procedure itself and post-surgery. Copious bleeding of the laceration is one problem that surgeons have to deal with during this type of surgical procedure. Recovery times for this technique vary between four to sixteen weeks as the incision is made in a very sensitive part of the palm of the hand. The possibility of recurrence should not be excluded. In addition, inability to see what lies beneath the carpal tunnel while cutting the associated ligament, gives rise to a risk in patients who have sustained hand injuries with consequences such as adhesions, nerve or tendon displacement and where there are undiagnosed malformations. Post-surgical pain prevents the patient from resuming activities, while those patients who manage in various ways to force hand movement in the first week are at risk of reopening the cut. Other negative consequences associated with the above method often include a scar that is not perfectly reabsorbed or insufficient incision of the ligament in question. Developments in this technique have led to a reduction in the size of the incisions, nevertheless, the fact that the incision of the volar wrist crease is always longitudinal means that there are still a high number of problems. A recent surgical technique envisages the use of endoscope and single and dual access techniques have also been developed. This particular procedure results in a small scar and much faster recovery: generally two/four weeks. In the case of single access from the wrist, the situation is fairly favourable. However the procedure incurs higher costs as it must be carried out by a surgeon with the assistance of at least one assistant, within a hospital facility equipped with operating theatre, not to mention the possible complications as set out in the literature.

US2007288043 discloses an instrument for use in minimally invasive carpal tunnel release, which include a cannula and a cutting member movable longitudinally within the cannula to advance a cutting blade along a longitudinal slot in the cannula to sever a transverse carpal ligament disposed over the slot. A dilating member is provided for creating a subligamentous space to accommodate the cannula and/or for removing adhered synovium from a lower surface of the ligament.

US2009048620 discloses a surgical knife and guide tool for performing carpal tunnel release surgery includes an elongated handle having a cutting head comprising an upper edge, a bottom edge, and a cutting edge therebetween, wherein the bottom edge comprises at least one glider extending from the bottom edge of the cutting head; and an elongated body having a longitudinal opening comprising a top portion having a first width for accepting the cutting head of the elongated handle and a bottom portion having a second width for accepting the at least one glider extending from the bottom edge of the cutting head, wherein the at least one glider controls longitudinal movement of the elongated handle.

WO 2010/030872 discloses the features of the preamble of claim 1.

### SUMMARY OF THE INVENTION

The invention is defined in the appended claims. The main scope of this invention is to provide a surgical instrument that eliminates, or at least limits, the aforementioned disadvantages.

In particular, said scope is achieved thanks to a surgical instrument that guides the surgeon in separation of the vascular/nerve tissue and anterograde incision of the transverse carpal ligament, without damage to the adjoining structures such as tendons, veins, the median nerve of the wrist and so forth. These and a number of other benefits, that will become clear following a detailed description of some of the execution methods of choice for the purposes of this invention, provided hereunder with reference to the annexed figures, provided as a non-exhaustive example, are achieved by a surgical instrument equipped with a special guide, having a blunt, slightly upward curving tip that allows a single surgical action to: efficiently separate the vascular/nerve structures so as to allow a clear view of the area to be operated; make an anterograde incision by means of a special retractable blade that slides within a channel of said guide, with high precision and safety, in the transverse carpal ligament without damaging other structures such as tendons, veins, the median nerve of the wrist and so forth; palpate in retrograde fashion the completeness of the incision made, in accordance with the scope of the present disclosure.

### BRIEF DESCRIPTION OF THE DRAWINGS

Fig. 1 perspective view from above of the instrument according to a first embodiment of this invention;
Fig. 2 perspective view from below of the instrument in figure 1;
Fig. 3 exploded view of the instrument in figure 1 and 2;
Fig. 4 detail of the front part of the instrument pursuant to this invention according to a first embodiment of figures 1 to 3;
Fig. 5 prospective, exploded view of the detail of the body and the blade of the instrument pursuant to this invention according to the first embodiment shown in figures 1 to 4;
Fig. 6 instrument insertion phase pursuant to this invention by means of incision of the volar surface of the wrist;
Fig. 7 incision phase of the distal edge of the transverse carpal ligament;
Fig. 8 incision phase of the proximal edge of the transverse carpal ligament;
Fig. 9 perspective view from above of the instrument according to a second, preferred embodiment of this invention;
Fig. 10 perspective view from below of the instrument pursuant to this invention according to the preferred embodiment as shown in figure 9;
Fig.11 perspective view from above of the instrument pursuant to this invention according to a third, preferred embodiment of this invention;
Fig. 12 perspective, partially disassembled view of the instrument in figure 11.

### DETAILED DESCRIPTION

With reference to the annexed figure, the surgical instrument pursuant to this invention shall now be described.

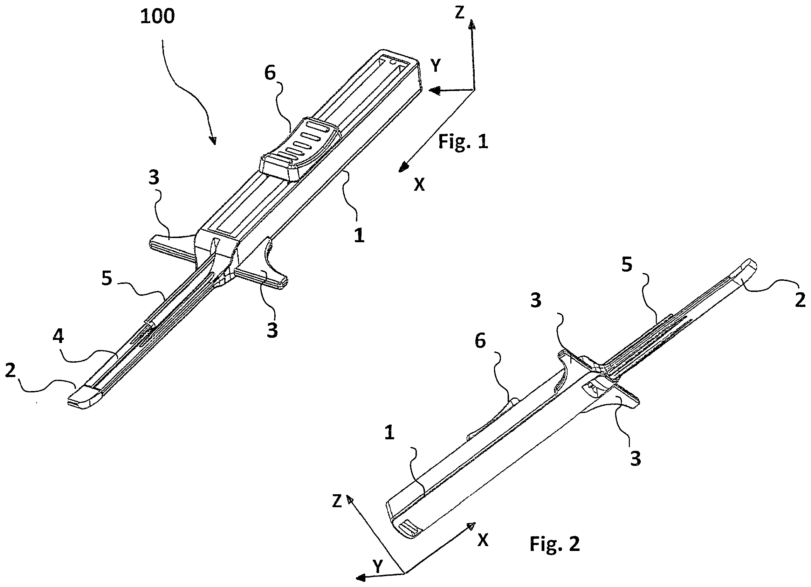

Figures 1 and 2 show the invention in all its parts.

Fig. 1 shows the surgical instrument 100, which includes a main body 1 from which a shaped guide 2, with a blunt, slightly upward-curving tip extends.The terms upper, inferior, high, low, front, rear and so forth refer to the Cartesian reference system shown in Figures 1 and 2. The blunt, upward-curving tip of the shaped guide 2 thus curves in the direction identified by the axis Z.The specific shape of the shaped guide 2 allows the vascular/nerve structures to be efficiently separated during use of the instrument so as to allow a clear view of the area to be operated.

Instrument 100 also includes a retractable blade 5.

In particular, as can be seen in the figures, the shaped guide 2 is essentially longitudinal in respect of the main body 1 of the instrument and includes, in an essentially axial position, a guide groove 4 that thus extends longitudinally along said shaped guide 2.

The retractable blade can move longitudinally in respect of the shaped guide 2 guided by said guide groove 4.

Since the movements of the blade 5 are precision-guided into the groove 4, when using the instrument the surgeon is able to cut in anterograde fashion, and with high precision and safety, the transverse carpal ligament without damaging the other structures such as tendons, veins, the median nerves of the wrist, and so forth.

It will also be possible to palpate, in retrograde fashion, completeness of the incision made.

The instrument 100 pursuant to this invention may advantageously include a further one or more handles 3 that are integral to the main body 1. The sliding blade 5 is firmly attached to the blade slider 5a. The blade slider 5a essentially comprises a hollow, essentially longitudinal member that can be firmly attached to said main body 1 of the instrument, and that has a front opening 5b. Activation means connected to the blade 5 for activation by the surgeon to allow said lame 5 to slide longitudinally may be present above said blade slider 5a.

In the first embodiment illustrated in figures 1 to 8, activation means 6 that can be activated by the surgeon include a button that is directly connected to said blade 5 and are linked in terms of sliding to said blade slider 5a.

Once the blade slider 5a is inserted in a special cavity 1a in the main body 1, the blade 5 is able to slide longitudinally in respect of the blade slider 5a and in respect of the main body 1, moving from a rest position in which said blade 5 is fully or partially retracted within said main body, to an operating position in which said blade 5 is extracted from said main body and exits from the latter further guided by the guide groove 4.

The function of guide groove 4 is thus to restrict transversal movement of the blade 5, allowing the latter to remain in axis with the shaped guide 2 during the longitudinal movements of the blade when in use, and decided by the surgeon by activating the activation device 6 of the blade 5.

For a better understanding of the system, it should be noted that the two handles 3 shown in the examples provided in the annexed figures are integral to the body 1 of the instrument 100, and that the blade slider 6 is in turn integral to said body 1 and that these are used by the surgeon to efficiently operate the instrument 100 using his/her fingers so as to release the transverse ligament of the median nerve, with the retracted blade, and subsequently resection it using the blade slider.

Fig. 2 once again shows instrument 100 pursuant to this invention from below with, in sequence: the shaped blade 2 that is capable of creating a path through the wrist tissue without damaging the tissue, the two small handles 3; detail of blade 5 (partial view), firmly housed in the structure of the blade slider 5a.

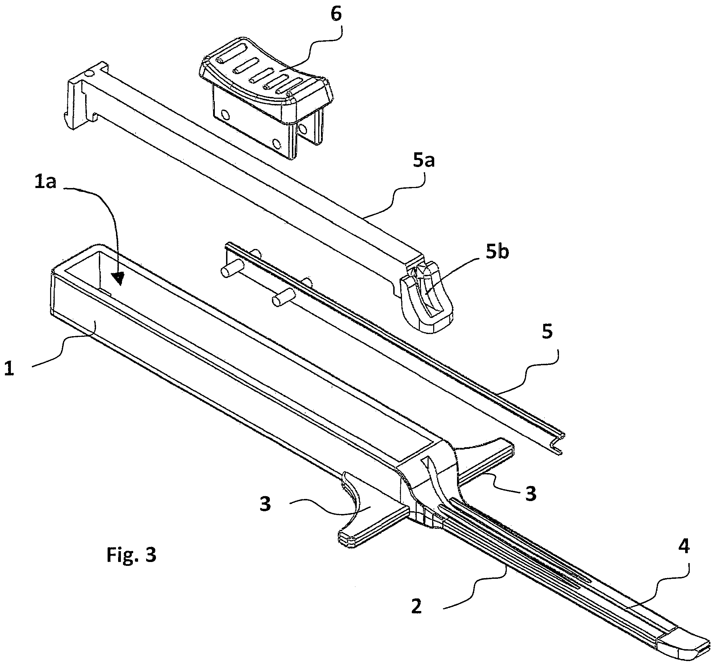

Figure 3 shows an exploded view of the invention. In Fig. 3, the instrument 100 with shaped guide 2, the handles 3; the grooving 4; the other parts separated from the body of instrument 1 including the special blade 5 and the blade slider 5a.

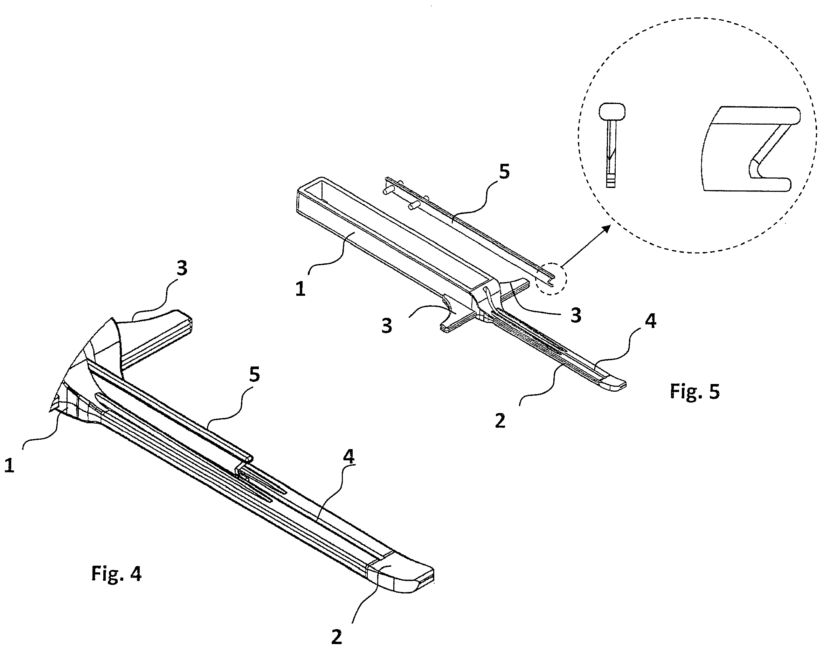

Figures 4 and 5 show details of the instrument pursuant to this invention. Fig. 4 shows the surgical instrument 100, with enlarged views of: the shaped guide 2, with a blunt, slightly upward-curving tip; the handles 3; the grooving 4, within which the special blade 5 slides. On the other hand, Fig. 5 shows the instrument 100 pursuant to this invention, inclusive of the shaped guide 2E, with a blunt, slightly upward-curving tip, the handles 3; the special blade 5, having been completely extracted from its housing. Magnification of Figure 5 shows the T-shape of the blade and the specific shape of the cutting edge that is able to capture and quickly and safely cut the transverse carpal ligament.

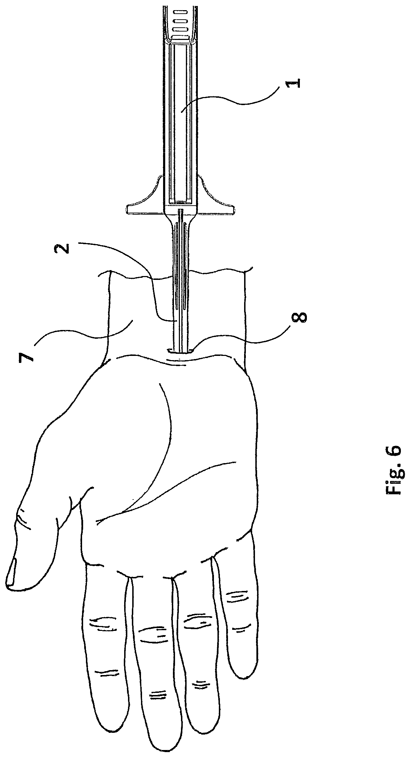

Figures 6 to 8 show certain stages of surgery that involve the use of the instrument pursuant to this invention.

In Fig. 6, the sliding blade 5 of the instrument 100 is fully retracted and inserted through an incision of the volar crease 8 of the wrist 7 and is used with its guide 2, which has a blunt, slightly upward-curving tip, to create a path through the tissue of the wrist 7 without any damage thereto.

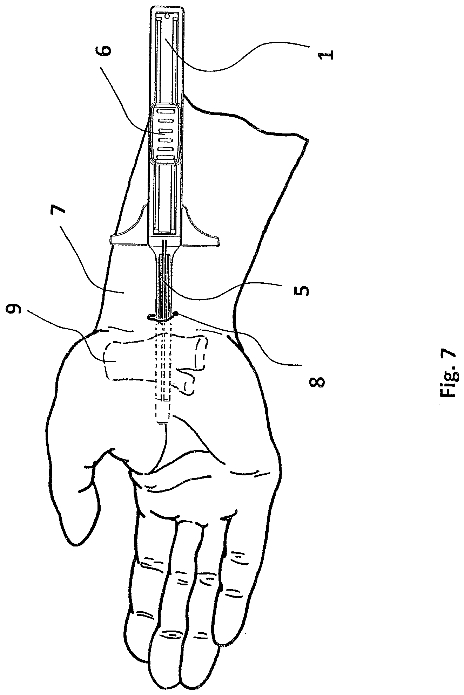

Figure 7 shows extraction of the blade from the body via activation of the blade slider 6 for resection of the distal part of the transverse carpal ligament 9.

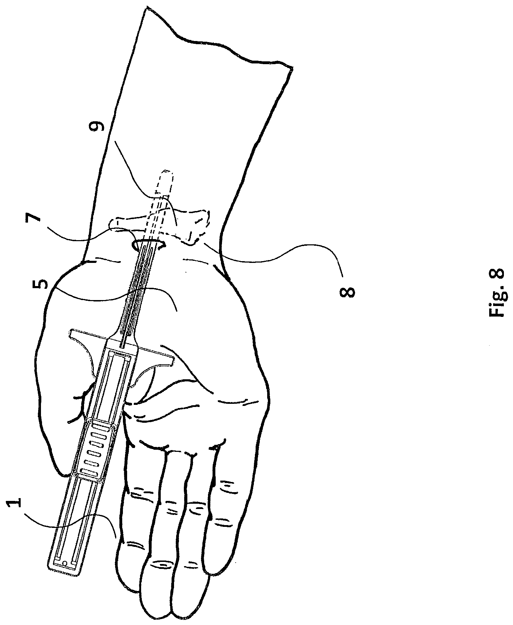

Figure 8 shows the use of the instrument pursuant to this invention for the resection of the proximal part of the transverse carpal ligament. In Fig. 8, instrument 100 is positioned in such a way as to allow it to be inserted in said access 8 used for the resection of the distal part but in an opposing position to the previously described position, so that incision of the proximal part of the transverse carpal ligament 9 may also take place with the special blade 5.

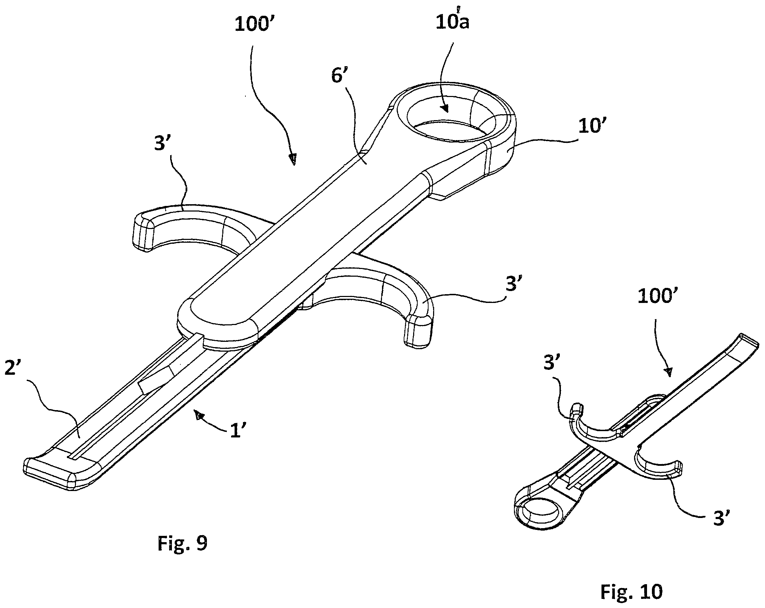

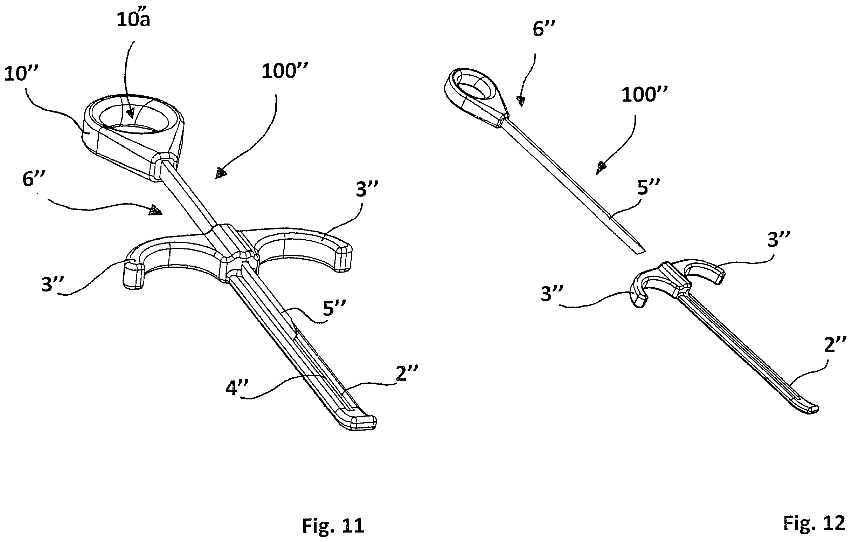

Figures 9 and 10 show a second preferred embodiment of the instrument pursuant to this invention, while figures 11 and 12 show a third preferred embodiment thereof.

The surgical instrument pursuant to this invention is equipped, as previously mentioned, with a special guide that has a blunt, slightly upward-curving tip, that allows the vascular/nerve structures to be efficiently separated in a single step so as to allow a clear view of the area to be operated and to, subsequently, safely cut with high precision, the transverse carpal ligament without damaging other structures such as tendons, veins, the median wrist nerve and so forth. All the variations of the instrument pursuant to this invention as are described herein include several essential characteristics for achieving the said technical result. In particular, it should be noted how the described embodiments vary in their secondary details, relating in particular to the shape of the gripping members for use by the surgeon, which in particular include the handles 3, 3' and 3" and a potential slot 10'a, 10"a that can be gripped by the surgeon's fingers.

Pursuant to this invention, the instrument 100, 100' and 100" includes a main body 1, 1' and 1", from which longitudinal guide members 2, 2' and 2" extend, said guide members having a longitudinal groove to guide the movement of the moving blade 5, 5' and 5" in respect of said guide members 2, 2' and 2", said guide members 2, 2' and 2" having a blunt, upward-curving tip.

It has thus been shown how the instrument pursuant to this invention achieves the envisaged scopes.

In particular, it has been shown how the instrument pursuant to this invention allows the transverse carpal ligament (9, 9B) to be cut in optimal fashion and without any troublesome post-surgical complications.

Thanks to the blunt guide with blunt, slightly upward-curving tip, used to efficiently separate the vascular/nerve structures, the instrument pursuant to this invention allows a clear view of the area to be operated consequently creating a path through the tissue of the wrist without causing damage thereto. Advantageously, the innovative instrument pursuant to this disclosure, can be constructed from various materials, thus making it suitable for single-use or sterile, without this compromising the correct functionality thereof.

The lateral movement of the blade is essentially restricted by the special form, and by interaction between the blade and the guide groove 4.

Indeed, in addition to preventing the upper part of the blade from cutting, its T-shape makes it more robust than a basic blade. The special shape of the blade also ensures that the transverse carpal ligament is fully and safely cut. The benefits of the instrument in question essentially comprise of the fact that correct operation of the instrument in accordance with the surgical technique for said instrument with a single access point in the volar part of the wrist, will result in quicker and completely safe incision of the transverse carpal ligament even in a basic medical clinic, without any need for referral to costly medical facilities such as hospitals. An entry point having been made by transverse incision results in a decidedly improves post surgery, allowing patients to resume their daily activities in less than two weeks, without pain or risk of the scar reopening and with optimal reabsorption of the scar, which is concealed with the crease itself. The additional benefits and characteristics of this utility model will be more or less clear to every sector technician from the description that follows and with the assistance of the annexed drawings, which are provided as practical examples of the invention and should not be deemed exhaustive.

The technique envisages the use of a local anaesthetic in the area surrounding the area of incision. The entire procedure is generally carried out without the need to reduce the blood flow to the limb.

After careful sterilisation of the surgical site, the wrist must be rested on a rigid, padded support and the hand must be allowed to fall on the operating surface so as to be almost perpendicular thereto (hyperextended wrist).

In addition to allowing a clear and deep view of the median nerve following incision, the procedure being non-invasive, this also allows the vascular structures of the palmar arch to be placed in an entirely different cutting plane than those of the transverse carpal ligament.

Incision of the skin is on the transversal crease of the wrist and extends for around 2.5 cm, slightly towards the ulnar side. With this entry-point, thanks to the position of the hand, the intermediate part and the distal part of the transverse carpal ligament will assume an almost vertical position; while its most proximal portion will be horizontal and more superficial. Fat is removed from the subcutaneous area and the area is opened up using two small blunt retractors thus exposing the superficial part of the ligament as far as the ulnar margin of the tendon of the long palmar muscle. Such repetition gives the surgeon confirmation that he/she has located the ulnar margin of the underlying median nerve.

It clearly not being possible to initiate incision at the distal or proximal end as suggested by classic techniques, a longitudinal incision is carefully made in the centre of the ligament with a scalpel. Stretching the two incised margins with Kelly forceps allows the area underlying the median nerve to be seen with ease. While the assistant lifts the skin with a mini-retractor, the instrument guide is inserted, through the incision thus made, in a proximal-distal direction, away from the ulnar part to reduce to a minimum any anomalous emergence of the motor branch due to thenar eminence. Due to its specific shape, the instrument guide will enter gently, its blunt end sliding below the deep face of the ligament while protecting the underlying median nerve. When the tip of the instrument can be felt under the skin of the palm of the hand (around 5 cm from the wrist), it has passed the distal margin of the ligament and is then blocked. The sharp part is then passed over the guide to, which it remains attached, thus allowing a completely safe and protected *"backwards and forwards"* movement, which allows full incision of the transverse carpal ligament. Where necessity demands, the same method can be utilised from the upwards incision thus resulting in a more complete section of carpal ligament of around 4/5 cm, both proximally and distally. At this stage, the median nerve is completely exposed to its deepest part and, using the guide instrument, retrograde palpation of all the tenotomy carried out is possible to ensure that no ligament band remains, in which case it can be readily removed.

The invention is not limited to the described or illustrated models and any expert can apply different variants in line with the scope of the claims. In practice, the execution details can nevertheless vary in a manner equivalent to the shape, dimensions, position of the parts and type of materials used, without in any case deviating from the adopted solution and thus remaining within the limits of the protection granted to this utility model patent.

## Claims

1. Surgical instrument (100, 100' and 100") comprising:
- a moving blade (5, 5', 5") lying in a first plane X - Z defined by a first axis X and a second axis Z perpendicular to said first axis X, and emerging perpendicularly with respect to a second plane X - Y, by defining an upward direction, wherein said second plane is defined by said first axis X and a third axis Y perpendicular to said first X and second axis Z and
- a main body (1 , 1' and 1"), from which
- a longitudinal guide member (2, 2' and 2") extends, according to said first axis X,
wherein
said guide member has a longitudinal groove (4, 4' and 4") according to said first axis X, to guide the movement of the moving blade (5, 5' and 5") in respect of said guide members (2, 2' and 2"), between an initial rest position and a second advanced cutting incision position, said guide member (2, 2' and 2") having a blunt, upward-curving tip emerging from said second plane X - Y and **characterized in that**
said moving blade (5) has a T-Shape and said surgical instrument (100, 100' and 100") further comprises a blade slider (5a) that is integral to said main body (1) and is suitable for guiding the longitudinal sliding movement of said moving blade (5) in respect of the main body (1) and within said groove (4) of said shaped guide (2) so that said moving blade (5) can be retracted within the blade slider (5a) during tissue detachment and palpation, without running the risk of unwanted resections.

2. Surgical instrument (100, 100' and 100") as claimed in the preceding claim, **characterised in that** it further comprises activating members (6. 6' and 6") that are connected to said blade (5, 5' and 5") that can be activated by the user to move said blade longitudinally (5, 5' and 5") in respect of the guide member (2, 2' and 2").

3. Surgical instrument (100, 100' and 100") as claimed in the preceding claim, **characterised in that** it further comprises gripping members for use by the surgeon (3, 3' and 3").

4. Surgical instrument (100, 100' and 100") as claimed in the preceding claim, **characterised in that** said gripping members comprise of at least a pair of handles (3, 3' and 3"), integral to said said body (1), that laterally protrude from the main body (1, 1' and 1").

5. Surgical instrument as claimed in one or more of the preceding claims, **characterised in that** it is made of resterilizable materials.

6. Surgical instrument (100, 100' and 100") as claimed in one or more of claims 1 to 4, **characterised in that** it is made of disposable materials.

## Patentansprüche

1. Chirurgisches Instrument (100, 100' und 100"), umfassend:
- eine bewegliche Klinge (5, 5', 5"), die in einer ersten Ebene X - Z liegt, die von einer ersten Achse X und einer zweiten Achse Z, normal zur ersten Achse X, definiert wird, und in Bezug auf eine zweite Ebene X - Y durch Definieren einer nach oben verlaufenden Richtung hervortritt, wobei die zweite Ebene von der ersten Achse X und einer dritten Achse Y, normal zu der ersten Achse X und der zweiten Achse Z, definiert ist, und
- einen Hauptkörper (1, 1' und 1"), aus dem sich
- ein längliches Leitelement (2, 2' und 2") gemäß der ersten Achse X erstreckt,
wobei
das Leitelement eine längliche Rille (4, 4' und 4") gemäß der ersten Achse X aufweist, um die Bewegung der beweglichen Klinge (5, 5' und 5") in Bezug auf die Leitelemente (2, 2' und 2") zwischen einer ruhenden Ausgangsposition und einer zweiten vorwärtsbewegten Schneideposition zu lenken, wobei das Leitelement (2, 2' und 2") eine stumpfe, sich nach oben hin krümmende Spitze aufweist, die aus der zweiten Ebene X - Y heraustritt,
und **dadurch gekennzeichnet, dass**
die bewegliche Klinge (5) eine T-Form aufweist und das chirurgische Instrument (100, 100' und 100") ferner einen Klingenschieber (5a) umfasst, der einstückig mit dem Hauptkörper (1) ausgebildet ist und zum Leiten der schiebenden Längsbewegung der beweglichen Klinge (5) in Bezug auf den Hauptkörper (1) und innerhalb der Rille (4) des geformten Leiters (2) geeignet ist, so dass die bewegliche Klinge (5) während des Abtrennens und der Palpation des Gewebes in den Klingenschieber (5a) eingezogen werden kann, ohne dabei unerwünschte Ektomien zu riskieren.

2. Chirurgisches Instrument (100, 100' und 100") nach einem der vorstehenden Ansprüche, **dadurch gekennzeichnet, dass** es ferner aktivierende Elemente (6, 6' und 6") umfasst, die mit der Klinge (5, 5' und 5") verbunden sind, die von dem Benutzer aktiviert werden können, um die Klinge in Bezug auf das Leitelement (2, 2' und 2") in Längsrichtung (5, 5' and 5") zu bewegen.

3. Chirurgisches Instrument (100, 10' und 100") nach einem der vorstehenden Ansprüche, **dadurch gekennzeichnet, dass** es ferner Greifelemente zur Verwendung durch den Chirurgen (3, 3' und 3") umfasst.

4. Chirurgisches Instrument (100, 100' und 100") nach einem der vorstehenden Ansprüche, **dadurch gekennzeichnet, dass** die Greifelemente zumindest ein Paar Griffe (3, 3' und 3") umfassen, die einstückig mit dem Körper (1) ausgebildet sind, die lateral aus dem Hauptkörper (1, 1' und 1") hervorstehen.

5. Chirurgisches Instrument nach einem oder mehreren der vorstehenden Ansprüche, **dadurch gekennzeichnet, dass** es aus resterilisierbaren Materialien hergestellt ist.

6. Chirurgisches Instrument (100, 100' und 100") nach einem oder mehreren der Ansprüche 1 bis 4, **dadurch gekennzeichnet, dass** es aus Einwegmaterialien hergestellt ist.

## Revendications

1. Instrument chirurgical (100, 100' et 100") comprenant :
- une lame mobile (5, 5', 5") reposant sur un premier plan X-Z défini par un premier axe X et un deuxième axe Z perpendiculaire audit premier axe X, et ressortant perpendiculairement par rapport au deuxième plan X -Y, en définissant une direction ascendante, dans lequel ledit deuxième plan est défini par ledit premier axe X et un troisième axe Y perpendiculaire auxdits premier X et deuxième axe Z et
- un corps principal (1, 1' et 1"), duquel
- un élément de guide longitudinal (2, 2' et 2") s'étend, selon ledit premier axe X,
dans lequel
ledit élément de guide a une rainure longitudinale (4, 4' et 4") selon ledit premier axe X, pour guider le mouvement de la lame mobile (5, 5' et 5") par rapport auxdits éléments de guide (2, 2' et 2"), entre une position initiale de repos et une deuxième position d'incision de coupure avancée, ledit élément de guide (2, 2' et 2") ayant une pointe courbée vers le haut émoussée venant dudit deuxième plan X-Y
et **caractérisé en ce que**
ladite lame mobile (5) est une forme de T et ledit instrument chirurgical (100, 100' et 100") comprend aussi un support de glissement de lame (5a) qui est intégré audit corps principal (1) et est apte à guider le mouvement glissant longitudinal de ladite lame mobile (5) par rapport au corps principal (1) et à l'intérieur de ladite rainure longitudinale (4) dudit guide modelé (2) de sorte que ladite lame mobile (5) puisse se rétracter à l'intérieur du support de glissement de lame (5a) pendant la séparation et la palpation du tissu, sans courir le risque de résections non désirées.

2. Instrument chirurgical (100, 100' et 100") selon la revendication précédente, **caractérisé en ce qu'**il comprend aussi des éléments d'activation (6, 6' et 6") qui sont reliés à ladite lame (5, 5' et 5") laquelle peut être activée par l'utilisateur pour déplacer longitudinalement ladite lame (5, 5' et 5") par rapport à l'élément de guide (2, 2' et 2").

3. Instrument chirurgical (100, 100' et 100") selon la revendication précédente, **caractérisé en ce qu'**il comprend aussi des éléments de préhension pour l'utilisation par le chirurgien (3, 3' et 3").

4. Instrument chirurgical (100, 100' et 100") selon la revendication précédente, **caractérisé en ce que** lesdits éléments de préhension sont composés d'au moins une paire de poignées (3, 3' et 3") intégrées audit corps (1), lesquelles dépassent latéralement le corps principal (1, 1' et 1").

5. Instrument chirurgical selon l'une ou plusieurs des revendications précédentes, **caractérisé en ce qu'**il est fabriqué avec des matériaux restérilisables.

6. Instrument chirurgical (100, 100' et 100") selon l'une ou plusieurs des revendications 1 à 4, **caractérisé en ce qu'**il est fabriqué avec des matériaux jetables.
